# EUROPÄISCHE PATENTSCHRIFT

(11) **EP 0 819 436 B1**
(45) Veröffentlichungstag und Bekanntmachung des Hinweises auf die Patenterteilung: **22.09.2004**
(21) Anmeldenummer: 97111128.1
(22) Anmeldetag: 03.07.1997
(51) Int. Cl.: A61K 45/06, A23L 1/304, A61P 19/10

(54) **Pharmazeutische und diätetische Zusammensetzungen umfassend Natrium-, Kalium-, Magnesium- und Calciumionen und Enzymaktivatoren**
Pharmaceutical and dietary composition containing sodium-, potassium-, magnesium- and calcium-ions and enzyme activators
Composition pharmaceutique et diététique contenant des ions de sodium, de potassium, de magnésium et de calcium et des activateurs d'enzymes

(30) Priorität: 19.07.1996 AT 130496
(43) Veröffentlichungstag der Anmeldung: 21.01.1998
(73) Patentinhaber: vis-vitalis Lizenz- und Handels GmbH, 5081 Anif (AT)
(72) Erfinder: Fuchs, Norbert,, A-5571 Mariapfarr (AT); Zelch, Norbert, Dr., A-5020 Salzburg (AT); Kössler, Peter, A-5571 Mariapfarr (AT); Loidl, Rupert, A-5571 Mariapfarr (AT)
(74) Vertreter: Alge, Daniel, Mag. Dr. rer.nat.

(56) Entgegenhaltungen:
- EP-A- 0 374 935
- WO-A-90/04402
- US-A- 4 812 303
- US-A- 5 424 331
- DATABASE WPI Section Ch, Week 9635 Derwent Publications Ltd., London, GB; Class A92, AN 96-349162 XP002086026 & JP 08 164 185 A (MORISHITA ROUSSEL KK) , 25.Juni 1996
- DATABASE WPI Section Ch, Week 9519 Derwent Publications Ltd., London, GB; Class B04, AN 95-144716 XP002086027 & JP 07 069 902 A (MEIJI SEIKA KAISHA LTD) , 14.März 1995
- DATABASE WPI Section Ch, Week 9125 Derwent Publications Ltd., London, GB; Class B04, AN 91-181418 XP002086028 & JP 03 109 329 A (IIZUKA K) , 9.Mai 1991

## Beschreibung

Die Erfindung betrifft pharmazeutische bzw. diätetische Zusammensetzungen, welche vorzugsweise zur Vorbeugung und zur Behandlung von Osteoporose eingesetzt werden können.

Der Begriff Osteoporose leitet sich von den griechischen Wörtern "osteon" bzw. "poros" ab und wurde Ende des 19. Jahrhunderts von Pathologen erstmals eingeführt.

Die Intention war, diese generalisierte Knochenerkrankung von den damals sehr häufig auftretenden Knochenerkrankungen wie Osteomalazie und Ostitis fibrosa cystica abzugrenzen. Erst der Endokrinologe Fuller Albright definierte die Osteoporose pathologisch eindeutig als einen Zustand "in which there is a lack of bone tissue but this bone tissue remains fully calcified". Heute wird Osteoporose meist als Verlust bzw. Verminderung von Knochensubstanz, -struktur und -funktion definiert, die zu erhöhter Frakturhäufigkeit führt, oder als eine generalisierte Skeletterkrankung, die durch niedrige Knochenmasse und eine gestörte Mikroarchitektur des Knochengewebes charakterisiert ist, wodurch es zu einer erhöhten Knochenbrüchigkeit und zu gesteigertem Frakturrisiko kommt.

Die verschiedenen Formen der Osteoporose lassen sich nach verschiedenen Kriterien einteilen, beispielsweise nach der Ätiologie, nach dem Verteilungsmuster der Osteoporose oder nach metabolischen Kriterien.

Nach der Ätiologie wird zwischen primären Osteoporosen unbekannter Genese und sekundären Osteoporosen mit endokrinen, metabolischen oder sonstigen bekannten Ursachen differenziert (Tab. 1).

Zu den primären Osteoporosen zählen die idiopathische Osteoporose, bei der, je nach Lebensabschnitt, in der sie auftritt, zwischen juveniler Osteoporose (bis 18 Jahre), idiopathischer Osteoporose im frühen Erwachsenenalter (bis 50 Jahre) unter schieden werden kann; die postklimakterische bzw. postmenopausische Osteoporose (50 bis 70 Jahre), die vermutlich durch den klimakterischen Östrogenabfall mitbedingt wird (daher auch die Bezeichnung Östrogenmangelosteoporose) und die multifaktoriell verursachte senile Osteoporose (ab 70 Jahre).

**Tabelle 1**

| Einteilung der Osteoporose nach der Ätiologie (mit Beispielen) | | |
|---|---|---|
| I. Primäre Osteoporose | | |
| | a) | Idiopathische Osteoporose |
| | b) | Postklimakterische Osteoporose (Typ I) |
| | c) | Senile Osteoporose (Typ II) |

| II. Sekundäre Osteoporose | | |
|---|---|---|
| | a) | Endokrinologisch/metabolisch verursachte Osteoporose (Cushing Syndrom, Hyperthyreose, Hypogonadismus, Hyperparathyreoidismus) |
| | b) | Iatrogen/medikamentös verursachte Osteoporose (Glucocorticoide, Heparin) |
| | c) | Im Rahmen komplexer Osteopathien (gastroenterologische Ursachen (Malnutrition, Malabsorption, Malassimilation), besondere Formen der renalen Osteopathie) |
| | d) | Im Rahmen neoplastischer Erkrankungen (multiples Myelom, Lympho- und Myeloproliferative Entartungen) |
| | e) | Hereditäre Bindegewebserkrankungen (Osteogenesis imperfecta, Marfan-Syndrom, Ehlers-Danlos Syndrom) |
| | f) | Reduktion der statischen Kräfte am Knochen (Bettruhe, Paraplegie, Schwerelosigkeit) |

Auch aufgrund ihres Verteilungsmusters läßt sich die Osteoporose in verschiedene Typen einteilen (Tab. 2).

**Tabelle 2**

| Verteilungsmuster der Osteoporose | | |
|---|---|---|
| A.: Generalisierte Osteoporose | | |
| | Typ I: | Spongiosa-betonter Knochenmasseverlust |
| | | Manifestation am Stammskelett durch Wirbelkörperfrakturen (Postklimakterische Osteoporose) |
| | Typ II: | Generalisierter Knochenmasseverlust (betrifft Spongiosa und Compacta), |
| | | Manifestation an langen Röhrenknochen durch Schenkelhalsfraktur, Radiusfraktur (senile Osteoporose). |

| B.: Lokalisierte, asymetrische Osteoporose (M. Sudeck) | | |
|---|---|---|
| | | |

Bei der Einteilung nach metabolischen Kriterien wird zwischen High-turn-over-Osteoporose (spontan einsetzender rascher Knochenverlust) und Low-turn-over-Osteoporose (langsamer Knochenverlust) unterschieden.

High-turn-over-Osteoporosen findet man vor allem bei Typ I bzw. postklimakterischer Osteoporose und bei der idiopathischen Osteoporose im frühen Erwachsenenalter. Ein rascher Knochenmineralverlust ("fast looser"; Knochenmineralverlust mit mehr als 3,5% innerhalb eines Jahres) leitet in der Regel die Aktivitätsphase der Osteoporose ein.

Zu den Low-turn-over-Osteoporosen zählen die Typ II bzw. Altersosteoporosen.

Über die Ursachen der Osteoporoseentstehung ist noch keine Klärung erzielt worden, es werden jedoch vor allem die Calciumtheorie, die Östrogenmangeltheorie und die zelluläre Theorie diskutiert.

Nach der Calciumtheorie liegen die Ursachen der Osteoporoseentstehung in einer Knochen- bzw. Calciumstoffwechselstörung, als Folge eines Calciummangels in der Ernährung, gastrointestinaler Malabsorption oder erhöhter Ausscheidung über die Nieren.

Die Östrogenmangeltheorie besagt, daß infolge der nach der Menopause auftretenden verminderten Östrogenproduktion bei Frauen mit individueller Disposition eine Osteoporose auftritt.

Bei der zellulären Theorie wird angenommen, daß die Osteoporose infolge einer Störung des Knochenaufbaus, bedingt durch eine Alterung der Osteoblasten auftritt. Die primäre Osteoporose mit verstärkter Osteoklastenaktivität soll durch eine Störung der negativen Rückkopplung zwischen der Calciumkonzentration im Serum und der Parathormon-Sezernierung aus den Nebenschilddrüsen verbunden sein.

Zur Therapie der Osteoporose werden derzeit im wesentlichen die folgenden therapeutischen Maßnahmen eingesetzt:
1. Therapeutika, die die Knochenresorption hemmen
2. Therapeutika, die die Knochenbildung stimulieren
3. Positivierung der Calciumbilanz
4. Symptomatische Schmerzbehandlung
5. Physikalische Therapie

Haupttherapieziel der Osteoporosebehandlung ist eine Verringerung der Frakturrate durch Stabilisierung bzw. Anheben des Skelettmineralgehaltes über die klinische Manifestations- bzw. Frakturgrenze. Zur Zeit gelten als Therapieerfolge bereits ein Sistieren des Skelettmineralgehaltes und das Erreichen von Schmerzfreiheit.

Als Therapeutika, die die Knochenresorption hemmen, werden am häufigsten Östrogene, Calcitonin und Biphosphonatderivate eingesetzt.

Östrogene können den rasch einsetzenden postmenopausalen Knochenverlust verhindern, aber nur so lange, wie die Zufuhr von Östrogenen erfolgt. Der Wirkungsmechanismus von Östrogenen ist bislang unbekannt. Fest steht nur, daß durch dieses Hormon die Osteoklastentätigkeit gehemmt wird. Die Protektion des Skeletts setzt eine mehrjährige (mindestens sieben Jahre) Östrogensubstitution voraus.

Zur Therapie der postmenopausalen Osteoporose werden hauptsächlich Östrogen/Gestagen-Kombinationspräparate eingesetzt, weil sich dadurch das östrogeninduzierte Endometriumkarzinomrisiko reduzieren läßt. Eine Östrogensubstitution erscheint vor allem im präklinischen Stadium der Typ I-Osteoporose sinnvoll, da hier der Knochenverlust der "Fast looser" gebremst werden kann.

Das Peptidhormon Calcitonin wird in den C-Zellen der Schilddrüse produziert und wirkt als Inhibitor der Osteoklastenfunktion, während die Osteoblasten nicht beeinflußt werden. Vorteilhaft ist die zentral-analgetische Wirkung des Calcitonins, auf die allerdings nur etwa 50 % der Patienten ansprechen. Empfohlen wird ein Therapiezeitraum von sechs Wochen, wobei die Behandlung als erfolglos abzubrechen ist, wenn innerhalb der ersten zwei Wochen keine Besserung eintritt. Der Nachteil einer Calcitoninbehandlung ist, daß dieses Neurohormon aufgrund seines Peptidcharakters nur parenteral verabreicht werden kann.

Biphosphonate hemmen die Skelettresorption und werden bei raschem Skelettverlust erfolgreich eingesetzt. Derivate des Pyrophosphats (Etidronsäure, Cladronsäure) bieten eine Reihe von Vorteilen, wie lange Halbwertszeit im Knochen, die eine compliance-fördernde, intermittierende Therapie erlaubt, und orale Wirksamkeit.

Als Therapeutika, die die Knochenbildung stimulieren, werden hauptsächlich Parathormon, Anabolika und Fluoride herangezogen.

Parathormon wäre zwar von seiner physiologischen Wirkung her geeignet, den Knochenaufbau zu stimulieren, jedoch liegen bis jetzt für eine kompetente Urteilsbildung zu wenige Erfahrungen vor.

Ein anaboles Steroid zur Behandlung der manifesten Osteoporose ist beispielsweise Nandrolondecanoat. Die Wirkungsweise von Nandrolondecanoat ist multifaktoriell. Es wird u.a. eine Stimulierung des periostalen Wachstums durch Positivierung der Stickstoffbilanz diskutiert. Insgesamt wird durch Nandrolondecanoat ein Anstieg der Knochenmasse sowie eine verbesserte Calciumresorption erreicht.

Fluoride fördern durch Stimulierung der Osteoblasten die Knochenneubildung. Die Osteoblasten bilden unter Fluorideinfluß in verstärktem Ausmaß eine Matrix aus Kollagen, in die dann Fluoridapatit eingelagert wird. Zu beachten ist, daß der Matrixaufbau dem Mineralisationsprozeß zeitlich vorauseilt, so daß der Knochen erst relativ spät wieder entsprechend belastbar ist. Die Fluoridtherapie wird sinnvollerweise durch Calcium und Vitamin D-Gaben ergänzt, da durch eine Kombinationstherapie der Einbau von Mineralsalzen in den Matrixknochen gefördert wird.

Der Calciumstoffwechsel nimmt bei der Ätiologie, Pathogenese und der Therapie der Osteoporose eine zentrale Stellung ein. Im menschlichen Organismus sind etwa 1000 bis 1500 g Calcium gespeichert, wovon sich etwa 99 % im Skelett befinden. Die Calciumaufnahme sollte täglich (je nach Lebensalter) zwischen 700 und 1000 mg betragen, um den täglichen Verlust auszugleichen.

In der JP 08/164185 A wird eine Bicarbonatlösung zur Behandlung von Azidosen beschrieben, welche Natrium-, Kalium-, Calcium-, Magnesium, Citrat- und Hydrogencarbonat-Ionen enthält und in einem Plastikbehälter mit einer Gas-Barriere-Eigenschaft verpackt ist.

In der US 4 812 303 A wird eine Tablette beschrieben, die im Wesentlichen aus Calciumcarbonat, Fumarsäure und Calciumlactat (als Exzipient) besteht, die eine Calciumzufuhr zur Verhinderung von Osteoporose darstellt.

In der WO 90/04402 A wird ein Osteoporosebehandlungsmittel beschrieben, welches alkalisierende Calciumionen umfasst, wobei dieses Mittel gegebenenfalls auch Magnesiumsalze beinhalten kann.

In der JP 07/69902 A wird eine Mischung aus einer Magnesiumquelle mit unverdaulichen Oligosacchariden u.a. Ballaststoffen beschrieben, mit welchen beispielsweise ein Magnesiummangel bei Osteoporose behandelt oder vorgebeugt werden kann.

In der EP 0 374 935 A1 wird ein Mittel zur Behandlung von Knochenschwund beschrieben, welches Kalksteinmehl, Dolomitmehl und Calciumhydrogenphosphat umfasst.

In der US 5 424 331 wird ein Mittel zur Behandlung oder Verhinderung von Osteoporose beschrieben, welches neben Süßholzwurzelextrakt auch Calcium-, Magnesium- und Zinksalze, sowie Beta-Carotin, Vitamin D und Vitamin E beinhaltet.

In der JP 03/109329 wird in Calcium-enthaltendes Pulver, welches gegebenenfalls noch Natrium, Phosphor, Kalium und Eisen enthalten kann, beschrieben.

Die EP 0 413 828 A1 offenbart eine feste Zusammensetzung, enthaltend aktiviertes Vitamin D₃, gemischt mit einer basischen Substanz und einem Exzipienten.

In der JP 60/193907 A wird eine zweischichtige Lotion mit Zinkoxid, Zink-p-phenol-sulfonat, Alkalimetallkarbonat und Wasser beschrieben.

Der vorliegenden Erfindung liegt die Aufgabenstellung zugrunde, eine völlig neue Behandlungsmöglichkeit der Osteoporose zur Verfügung zu stellen, welche nicht nur die symptomatische Behandlung oder Stabilisierung von osteoporosepatienten ermöglicht, sondern vor allem den Ursachen der Osteoporose entgegenwirkt.

Gelöst wird diese Aufgabe durch eine pharmazeutische oder diätetische Zusammensetzung umfassend
- Natrium-, Kalium-, Magnesium- und Calcium-Ionen, wobei zumindest Natrium und Kalium als Carbonate und/oder Bicarbonate vorliegen, und
- für den menschlichen Körper essentielle Mengen- und Spurenelemente, ausgewählt aus Li-, Sr-, Zn-, Fe-, Mn-, Cu-, Cr-, Mo-, Se-, F-Ionen, Vitaminen, vorzugsweise Vitamin C, K und D₃, sowie Mischungen derselben,
wobei das Masseverhältnis von K : Ca : Na : Mg 0,5-20 : 0,2-16 : 0,1-10 : 1 beträgt, die Zusammensetzung wenigstens 40 mmol an alkalischen oder alkalisierenden Ionen enthält und die in Wasser gelöste Zusammensetzung einen pH-Wert von ≥ 8,0 aufweist.

Die Wirkungsweise dieses Präparates beruht auf alternativen Denkansätzen zur Osteoporoseentstehung, nämlich der Osteoporoseentstehung aus der Sicht des Säure-Basen-Haushaltes und dem System der Grundregulation im Hinblick auf die Osteoporoseentstehung:

Zur Aufrechterhaltung eines, je nach Kompartiment unterschiedlichen, konstanten pH-Wertes stehen dem Körper mehrere Puffersysteme (Blut, Bindegewebe, Niere, Lunge, IZR, EZR) zur Verfügung. Jahrelange Fehlernährung, suboptimale Mikronährstoffversorgung sowie physische und psychische Stressoren erschöpfen das Neutralisationsvermögen der körpereigenen Regulationssysteme, wodurch eine Links- (Azidosen) bzw. Rechtsverschiebung (Alkalosen) im Säure-Basen-Gleichgewicht induziert wird.

Die Folgen einer Linksverschiebung des Säure-Basen-Gleichgewichts sind durch Säuren hervorgerufene Bindegewebsschäden ("Gewebsazidosen"), die als wesentliche Ursachen für Zivilisationskrankheiten wie Herzinfarkt, Osteoporose, Rheuma und Gicht gelten.

Zu den Verursachern dieser "Gewebeübersäuerung" zählen nichtflüchtige Stoffwechselmetabolite, die aus säurebildenden Nahrungsbestandteilen (raffinierte Kohlenhydrate, tierische Proteine, Kaffee), bei gleichzeitig alkaliarmer Ernährung, gebildet werden. Die durch intermediäre Stoffwechselprozesse gebildeten Säuren werden vom Blut aufgenommen, neutralisiert und über pulmonale Abatmung (CO₂) und renale Exkretion aus dem Körper eliminiert.

Die Neutralisation von Säuren über das Blutpuffersystem ist allerdings nur im Rahmen seiner Pufferkapazität möglich (enger Arbeits-pH!). Säureüberschüsse werden ins Interstitium abgeführt gepuffert und, bis zum weiteren Abtransport über Blut (bei freien Pufferkapazitäten) und Nieren, zwischengelagert. Eine direkte Ausscheidung von Substanzen aus dem Bindegewebe ist aufgrund fehlender direkter Ausscheidungsmöglichkeiten nicht möglich.

Azidotische Stoffwechsellagen führen zu einem Abzug von alkalischen Salzen aus dem Körper. Die Folge ist, daß die Mobilisierung von Calcium, Magnesium und Phosphat (alkalische Salze) aus den Knochen zunimmt, was insgesamt zu einer Demineralisierung des Skeletts führt.

Selbst eine ausreichende Calciumsupplementation kann den Demineralisationsprozeß nicht stoppen, da das resorbierte Calcium sofort zur Neutralisation der Säuren abgezogen und so nie für den Einbau in das Skelett verfügbar wird.

Als weiterer Aspekt bei der Entstehung von aziden Stoffwechsellagen mit verstärkter Calciummobilisierung aus den Knochen wird eine Akkumulation von Homotoxinen (Säurecharakter) bei menopausalen Frauen nach der Homotoxinlehre von Reckeweg diskutiert.

Das System der Grundregulation wird definiert (nach Pischinger) als Funktionseinheit der Gefäßendstrombahn, der Bindegewebszellen und der vegetativ-nervalen Endformation. Das gemeinsame Wirk- und Informationsprinzip dieser Trias ist die extrazelluläre Flüssigkeit. Angeschlossen sind die Lymphgefäße und Lymphorgane. Das in die extrazelluläre Flüssigkeit eingebettete Bindegewebe ist somit das größte und einzige, den ganzen Körper durchziehende System, das mit jeder Körperzelle in direktem Kontakt steht.

Biochemisch bildet die Grundsubstanz (extrazelluläre Matrix) ein Netzwerk (Molekularsieb) bestehend aus Polysacchariden, die z.T. an Protein gebunden sind (Proteoglykane und Glykosaminoglykane, PG/GAGs) sowie Struktur- und Vernetzungsproteinen (Kollagen, Elastin, Fibrinonektin, u. a.). Als funktionelle Aspekte der extrazellulären Matrix gelten Transmitter-, Speicher- und Regulationsaufgaben.

Die Transmission von Molekülen steht in engem Zusammenhang mit der Molekularsiebstruktur der Grundsubstanz wobei die Porengröße des "Filters" von der Konzentration und Qualität der gelösten Proteoglykane, deren Molekulargewicht, dem pH-Wert der Lösung und den anwesenden Elektrolyten (gelöste Mengen- und Spurenelemente) im betreffenden Gewebsbereich abhängig ist.

Endogene Fehlbildungen (erbliche oder altersbedingte Ansammlungen genetischer Fehler und Irrtümer der zellulären Kontrollmechanismen) oder exogene Faktoren (Umweltbelastungen, chemische Radikale, Fehlernährung, Streß, virale und bakterielle Infektionen, Strahlung, alkylierende Substanzen) beeinflussen sämtliche Synthese- und Stoffwechselschritte der Strukturkomponenten der Grundsubstanz (Einschränkung der Funktion der Grundsubstanz!), wodurch u.a. die große Zahl an Bindegewebs-, Gefäß-, Knorpel- und Knochenerkrankungen zustande kommt.

Mit zunehmendem Alter treten vermehrt unphysiologische Veränderungen der Grundsubstanz auf ("Verschlackung"). Bindung von Schwermetallionen, Antigen-Antikörperkomplexen und Defektproteinen an PG/GAGs, nicht-enzymatische Glykosilierungen von zukkerhältigen Substanzen und Verdickung der Basalmembran sind Kennzeichen dieser Verschlackung und führen insgesamt zu einer erschwerten zellulären Ver- und Entsorgung sowie zu Gewebshypoxien, die ihrerseits wiederum die Entstehung von generellen Azidosen begünstigen.

Insgesamt gesehen bewirken Verschlackung und auftretende Azidosen einerseits eine verminderte Nährstoffversorgung der Knochenzellen (durch unphysiologische Strukturveränderungen der Grundsubstanz) und andererseits einen Entzug von basischen Ionen aus dem Skelett (zur Kompensation azidotischer Tendenzen).

Die Zusammensetzungen der vorliegenden Erfindung erreichen eine deutliche Verbesserung der Knochenmineralisation durch eine ausgewogene Zufuhr der wichtigen Elemente Natrium, Kalium, Calcium und Magnesium, sowie gegebenenfalls weiterer essentieller Makround Mikroelemente über einen längeren Zeitraum. Entscheidend dabei ist, daß die genannten Elektrolyte an alkalische bzw. alkalisierende Anionen, wie Carbonate, Bicarbonate, Zitrate, Gluconate, Tartrate oder Lactate, gebunden vorliegen.

Somit wird eine Stärkung der Pufferkapazitäten extra- und intrazellulärer Räume, insbesondere des Bindegewebes, durch basische Kationen und Anionen (also durch einen Ausgleich azidotischer Tendenzen durch gezielten Einsatz alkalischer Verbindungen) ermöglicht, wobei die Filter- bzw. Molekularsiebfunktion der "Grundsubstanz" und damit eine erhöhte Transmission von Mikronährstoffen regeneriert wird.

Die erfindungsgemäße Zusammensetzung umfaßt vorzugsweise weitere Stoffe, beispielsweise Gerüststoffe, vorzugsweise auf Basis von Silizium, und/oder Zusatz- oder Hilfsstoffe, vorzugsweise Kohlehydrate und/oder Aromastoffe.

Diese Stoffe dienen sowohl zur Verbesserung der Wirkung der Zusammensetzung als auch zur Fertigstellung eines verkaufsfertigen Produktes. Als Zusatzstoffe kommen u.a. Kohlehydrate, wie Fruktose, Saccharose oder Stärke, natürliche und natur-identische Aromen oder andere Geschmackstoffe in Betracht.

Als Enzymaktivatoren werden die Mengen- und Spurenelemente eingesetzt, welche für den menschlichen Körper essentiell sind oder speziell bei Osteoporose-Erscheinungen benötigt werden, also Li-, Sr-, Zn-, Fe-, Mn-, Cu-, Cr-, Mo-, Se-, F-Ionen, Vitamine, vorzugsweise Vitamin C, K und D₃, sowie Mischungen dieser Enzymaktivatoren.

Die bevorzugten Anionen, welche in der erfindungsgemäßen Zusammensetzung verwendet werden können sind z.B. Carbonat, Hydrogencarbonat, Glycerophosphat, Pyrophosphat, Gluconat, Citrat, Ascorbat, Lactat, Molybdat, Chlorid und Phosphat.

Bei der Bestimmung des bevorzugten Mengenverhältnisses sind neben den bekannten Tagesbedarf-Werten bzw. -Verhältnissen auch auf den individuellen Bedarf des Patienten oder (bei der Prophylaxe) des Gesunden abzuzielen. Daher enthält die erfindungsgemäße Zusammensetzung Kalium, Calcium, Natrium und Magnesium in einem Masseverhältnis von 0,5-20 : 0,2-16 : 0,1-10 : 1, insbesondere von 1-10 : 0,5-8 : 0,3-5 : 1, wobei die genannten Elektrolyte an alkalische oder alkalisierende Anionen (in einer Gesamtmenge von mindestens 40 mmol) gebunden vorliegen.

Die zu verabreichende Dosis sollte möglichst nahe an dem individuellen Bedarf des jeweiligen Patienten liegen. In der Regel wird die Behandlung und die Vorbeugung mit der durchschnittlichen Tagesbedarf-Dosis durchgeführt, welche jedoch im Bedarfsfall gegebenenfalls über- bzw. unterschritten werden kann (z.B um den Faktor 3 bzw. 1/3).

Bei normalem Tagesbedarf ist in der Regel ein Masseverhältnis K : Ca : Na : Mg von etwa 5 : 2,5 : 1,5 : 1 ausreichend.

Die erfindungsgemäße Zusammensetzung muß in Lösung einen alkalischen pH-Wert aufweisen, welcher bevorzugterweise im Bereich von 7,5 bis 9,5, vorzugsweise von 8,9 bis 9,0, liegt.

Das Masseverhältnis (% w/w) der Kohlehydrate gemäß der vorliegenden Zusammensetzung beträgt vorzugsweise weniger als 40%, insbesondere weniger als 20% oder sogar weniger als 1%.

Beim Einsatz der erfindungsgemäßen Zusammensetzung als Medikament sollte sie in einer pharmazeutisch verabreichbaren Formulierung vorliegen, wobei die Verabreichungsformulierung vorzugsweise als orale oder parenterale Verabreichungsform ausgeführt ist. Nähere Einzelheiten zu geeigneten pharmazeutischen Formulierungsstoffe (abhängig von der Verabreichungs- bzw. Dosisform) sind dem Fachmann auf dem vorliegenden Gebiet geläufig und können auch aus der Pharmakopöe entnommen werden.

Die erfindungsgemäße Zusammensetzung kann auch als Nahrungsmittel oder als Zusatz für ein Nahrungsmittel verwendet werden, wobei ebenfalls bekannte lebensmitteltechnisch geeignete Zusatzoder Trägerstoffe mit der Zusammensetzung zu einer lebensmitteltechnisch geeigneten Formulierung kombiniert werden können.

Ein weiterer Aspekt der vorliegenden Erfindung betrifft die erfindungsgemäße Zusammensetzung als Arzneimittel.

Weiters betrifft die vorliegende Erfindung die Verwendung der erfindungsgemäßen Zusammensetzung zur Herstellung einer Präparation zur Vorbeugung und Behandlung von azidotischen Erkrankungen.

Gegenstand der Erfindung ist selbstverständlich auch die Verwendung der erfindungsgemäßen Zusammensetzung zur Herstellung einer Präparation zur Vorbeugung und Behandlung von Osteoporose, und zwar aller Arten der Osteoporose (s.Tabellen 1 und 2), wobei insbesondere die weit verbreitete primäre Osteoporose bzw. die generalisierte Osteoporose im Vordergrund stehen sowie die Stimulierung der Knochenmineralisation und des Aufbaus der organischen Knochenmatrix nach Knochenfrakturen.

Weiters kann die erfindungsgemäße Zusammensetzung zur Herstellung einer Präparation zur Regeneration des Ionenhaushaltes, insbesondere in Bindegewebszellen, sowie zur Herstellung einer Präparation zur Stabilisierung und Verbesserung der Knochendichte, insbesondere der Knochenmasse und der Knochenmineraldichte, verwendet werden.

Die Erfindung wird durch die nachfolgenden Beispiel, auf die sie jedoch nicht eingeschränkt ist, näher erläutert.

### Beispiele

### 1. Diagnostik

### 1.1. Röntgendiagnostik

Typische röntgenologische Veränderungen an der Wirbelsäule bei Osteoporose sind neben einer vermehrten Strahlendurchlässigkeit mit Erweiterung der Trabekelabstände, Verformungen der Wirbel als Keil-, Platt- und Fischwirbel. Die Röntgenuntersuchung eignet sich allerdings für die Frühdiagnose der Osteoporose nicht, da erst bei einem Knochenmineralverlust von mehr als 30-40 % eine Osteoporose radiologisch deutlich erkennbar wird. Diese Methode wird daher nur noch zur Diagnose der manifesten Osteoporose mit schon eingetretenen Frakturen verwendet.

### 1.2. Knochendichtemessung (Osteodensitometrie)

### 1.2.1. Quantitative Computertomographie (QCT)

Die quantitative Computertomographie (QCT) eignet sich sehr gut zur selektiven Dichtebestimmung von Spongiosa und Compacta. **Prinzip:** Mit Hilfe von spezieller Software und Kalibrierungsphantomen wird aus der Abschwächung von Röntgenstrahlung in einem definierten Raum des untersuchten Körpers der Knochenmineralgehalt bestimmt.

| | |
|---|---|
| Meßdauer | 10 bis 20 Minuten |
| Strahlenbelastung | 50 bis 500 mrem |
| Reproduktionsfehler | 3 bis 10 % |

Meßfehler ergeben sich durch den Fettgehalt des Knochenmarks im sog. fetthaltigen oder gelben Knochenmark. Die Reproduzierbarkeit der Ergebnisse beim QCT hängt entscheidend davon ab, ob bei Wiederholungsuntersuchungen das Meßorgan exakt wieder getroffen wird. Verschiebungen von 1 mm können bereits Fehler von 1 % hervorrufen

### 1.2.2. Periphere quantitative Computertomographie (pQCT)

**Prinzip:** Der Knochen wird mit Hilfe einer automatischen Formerkennung analysiert und das Hydroxyapatitäquivalent (HE) im Volumen bestimmt, wobei es möglich ist, das HE in Compacta und Spongiosa getrennt zu ermitteln. Da es sich bei pathologischmetabolischen Knochenveränderungen meist um generalisierte Systemerkrankungen handelt, ist eine Messung an Referenzpunkten, an denen kaum sekundäre Veränderungen vorliegen, notwendig.

### 1.2.3. Ein-Energien-Photonen-Absorptiometrie (Single-Photon-Absorptiometry, SPA)

Zur Bestimmung des lokalen Knochenmineralgehaltes im peripheren Skelett (Unterarm, Calcaneus) wird die SPA verwendet. **Prinzip:** Von einer Strahlenquelle (¹²⁵Jod) wird monoenergetische Gammastrahlung ausgesendet, die den Körper des Patienten durchdringt. Aus der Intensitätsabschwächung des Photonenstrahls kann direkt auf den Mineralsalzgehalt des Knochens geschlossen werden.

| | |
|---|---|
| Untersuchungsdauer | 15 bis 20 Minuten |
| Strahlenbelastung | 2 bis 5 mrem |
| Reproduktionsfehler | 2 % |

Meßfehler ergeben sich hier aus der Absorption der Strahlen in den Weichteilen.

### 1.2.4. Zwei-Energien-Photonen-Absorptiometrie (Dual-Photon-Absorptiometry, DPA)

Die DPA wird vorwiegend zur Messung des Knochenmineralgehaltes des mehr spongiösen Knochens der Lendenwirbelsäule und des proximalen Femur eingesetzt. **Prinzip:** Als Strahlenquelle bei dieser Methode dienen radioaktive Substanzen (vorwiegend ¹³⁵Gadolinium), die monochromatische Strahlung mit zwei Energiemaxima emittieren. Diese Strahlung wird beim Durchgang durch Gewebe abgeschwächt, wobei die Strahlungsabschwächung vornehmlich vom Knochenmineralgehalt bestimmt wird.

Bedingt durch die unterschiedliche Absorptionsrate der zwei monoenergetischen Photonenenergien im Weichteilgewebe und im Knochen, kann der Knochenmineralsalzgehalt ohne Weichteilfehler ermittelt werden.

| | |
|---|---|
| Untersuchungsdauer | 30 Minuten |
| Strahlenbelastung | 2 bis 3 mrem |
| Reproduktionsfehler | 2% |

Eine spezielle Fehlerquelle dieses Verfahrens liegt in der Erschöpfüng der Strahlenquelle, wodurch sich die ausgesandte Strahlenqualitat verändert.

### 1.2.5. Zwei-Energien-Absorptiometrie mit Röntgenstrahlen (Dual-Energy-X-Ray-Absorptiometry, DXA)

Andere Bezeichnungen für dieses Verfahren sind auch DPX (Dual Photon X-Ray Absorptiometry), DEXA (Dual Energy X-Ray Absorptiometry) oder QDR (Quantitative Digitale Radiographie). **Prinzip:** Die DXA, eine Weiterentwicklung der DPA, benutzt anstelle von radioaktiven Substanzen eine Röntgenröhre als Strahlenquelle, die analog der DPA, mit zwei Strahlenmaxima arbeitet. Die Knochenmasse wird über das Hydroxyapatitäquivalent (HE) in der Lendenwirbelsäule bestimmt.

| | |
|---|---|
| Untersuchungsdauer | 5 Minuten |
| Strahlenbelastung | 0,5 bis 3 mrem |
| Reproduktionsfehler | 1 % |

Fehlbestimmungen des Knochenmineralgehaltes können, als Folge von Veränderungen an Wirbelkörpern, wie eine Skoliose, schwere degenerative Veränderungen oder auch nach Wirbelbrüchen auftreten. Als weitere Meßfehlerquellen gelten - vorausgesetzt diese Strukturen liegen im Meßfeld - auftretende Spondylarthrosen, Spondylophyten, Verkalkungen in Lymphknoten oder Gefäßen und Röntgenkontrastmittel, die sich aufgrund anderer Untersuchungen im Körper befinden. Zur Reduktion dieser Fehler ist bei der DXA-Messung immer zusätzlich ein konventionelles Röntgenbild erforderlich. Unter Berücksichtigung der Strahlenbelastung gilt die DXA als Methode der Wahl, insbesondere bei umfangreichen und wiederholten Messungen bei postmenopausalen Frauen, für orientierende Serienuntersuchungen sowie zur Verlaufs- und Therapiekontrolle.

### 2. Bereiten der Zusammensetzung:

Eine erfindungsgemäße Zusammensetzung wurde aus Natrium-(vorwiegend als Hydrogencarbonat), Kalium- (als Carbonat und Citrat), Magnesium- (vorwiegend als Carbonat) und Calcium-Salzen (vorwiegend als Carbonat und Chlorid) durch Mischen bereitet, welche 800 mg K, 400 mg Ca, 250 mg Na und 150 mg Mg umfaßte.

Die Zutaten waren die folgenden: Fructose, Stärke, Kaliumcarbonat, Magnesiumcitrat, natürliche und natur-identische Aromen, Calciumcarbonat, Natriumhydrogencarbonat, Kieselerde, Calciumchlorid, Magnesiumcarbonat, Kaliumcitrat, Calciumglycerophosphat, Natriumpyrophosphat, Natriumascorbat, Eisengluconat, Mangangluconat, Zinkgluconat, Selenhefe, Strontiumlactat, Kupfergluconat, Lithiumcarbonat, Natriumfluormonophosphat, Chrom-IIIchlorid-hexahydrat, Natriummolybdat, Phytomenadion, Cholecalciferol.

Daher enthielt diese Zusammensetzung die folgenden Mengen- und Spurenelemente:

| | | |
|---|---|---|
| Silizium: | | 129,90 mg |
| Chlorid | | 120,40 mg |
| Phosphor | | 35,75 mg |
| Mangan | | 5,55 mg |
| Eisen | | 5,55 mg |
| Zink | | 5,55 mg |
| Strontium | | 5,55 mg |
| Lithium | | 1,10 mg |
| Kupfer | | 1,10 mg |
| Fluor | | 550,00 µg |
| Chrom | | 111,00 µg |
| Molybdän | | 111,00 µg |
| Selen | | 55,50 µg |
| Vitamine | C | 55,60 mg |
| | K | 30,00 µg |
| | D₃ | 3,00 µg |

### 3. Verabreichen des erfindungsgemäßen Präparates

Das in Beispiel 2 hergestellte Präparat wurde zweimal täglich in 1/4 l Wasser oder Gemüsesaft (nicht CO₂-haltiges Mineralwasser oder Fruchtsäfte und andere säurehaltige Getränke) eingerührt und nach den Mahlzeiten eingenommen.

Zur Untersuchung der Wirksamkeit des erfindungsgemäßen Mittels zur Behandlung postklimakterischer Osteoporose wurden die Patientinnen in mehrere Untersuchungsgruppen unterteilt:
Gruppe 1: ohne Therapie (15 Patientinnen)
Gruppe 2: orale Hormontherapie (15 Patientinnen)
Gruppe 3: Behandlung mit dem erfindungsgemäßen Mittel (20 Patientinnen)
Gruppe 4: Behandlung mit dem erfindungsgemäßen Mittel plus Behandlung mit Hormonen (18 Patientinnen)

Die Gruppe 1 stellt dabei den Vergleichswert ohne jede Behandlung dar; die Gruppe 2 zeigt eine Behandlung gemäß dem Stand der Technik; Gruppe 3 zeigt die Behandlung mit dem erfindungsgemäßen Präparat; und Gruppe 4 die Kombination des erfindungsgemäßen Präparats mit einer Behandlung gemäß dem Stand der Technik.

Die Ergebnisse sind in den Tabellen 3 bis 6 aufgelistet und zeigen deutlich, daß mit der erfindungsgemäßen Zusammensetzung überraschenderweise die besten Werte bei der Veränderung der Knochendichte (ermittelt mittels DXA-Verfahren an der Wirbelsäule bzw. an Oberschenkel/Hüfte) im Behandlungszeitraum zwischen erster und zweiter Messung (ca. 12 Monate) erzielt werden konnte (im Schnitt +2,88 %). Mit der oralen Hormontherapie gemäß dem Stand der Technik konnte im Schnitt + 1,85 % an Knochendichte gewonnen werden, mit der Kombination von Hormontherapie mit erfindungsgemäßer Zusammensetzung immerhin noch +1,66 %. Im Vergleichszeitraum nahm die Knochendichte bei unbehandelten Patientinnen dagegen um 3,47 % ab.

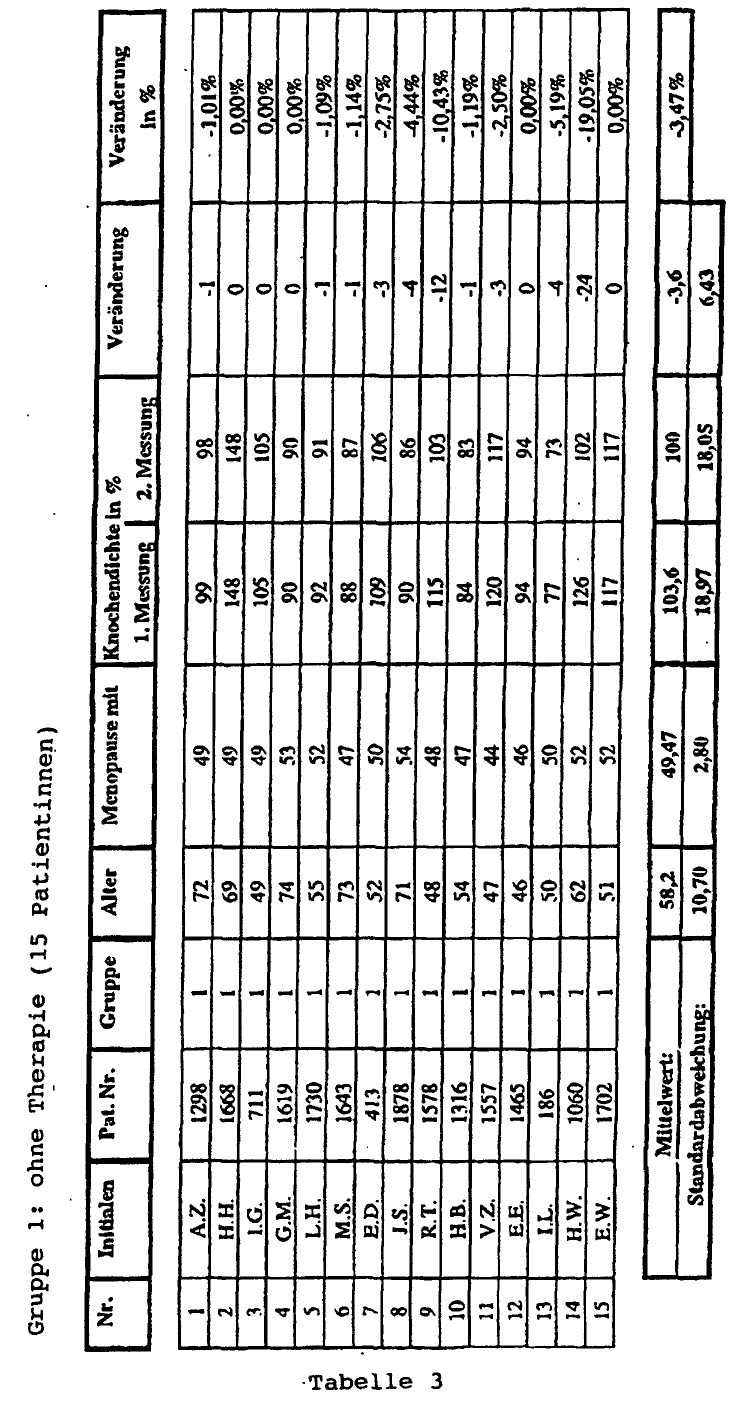

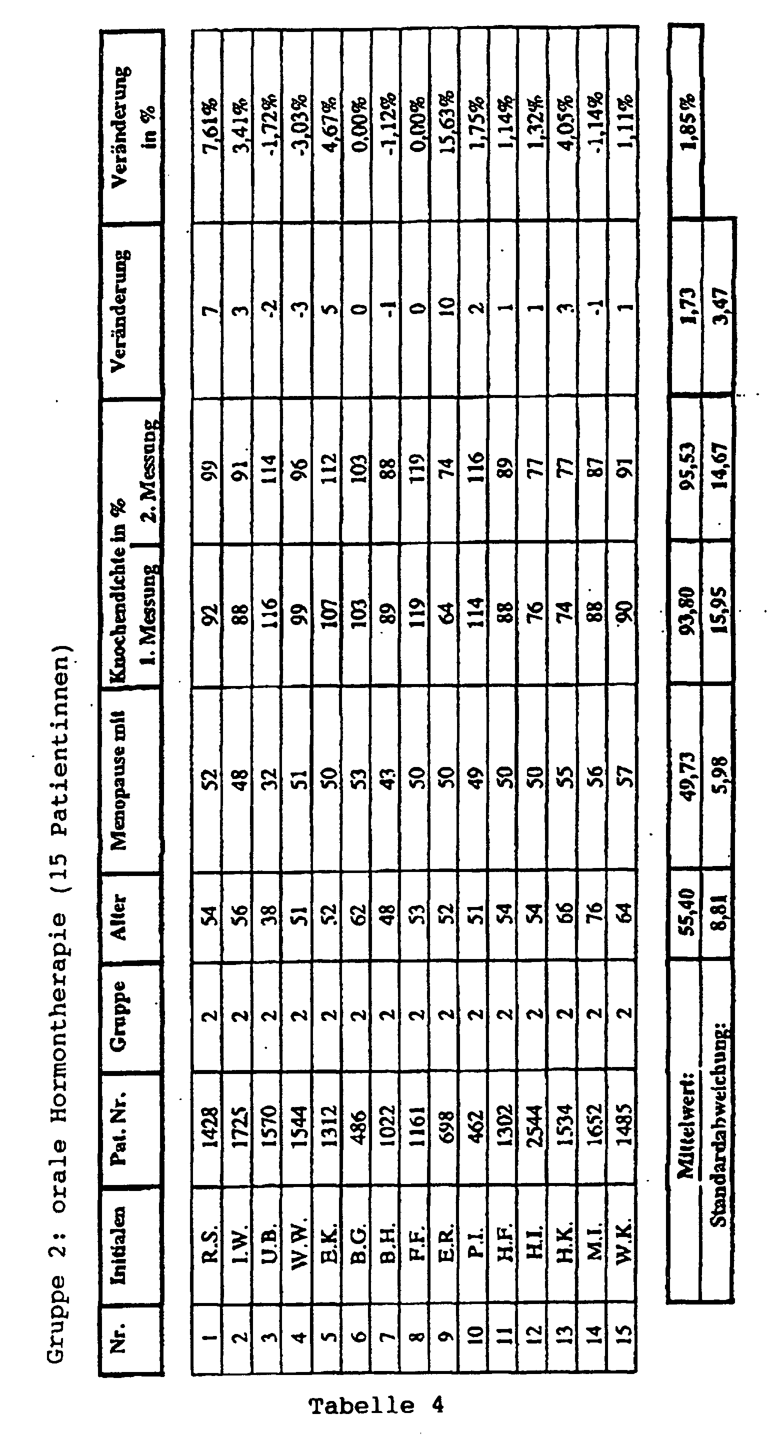

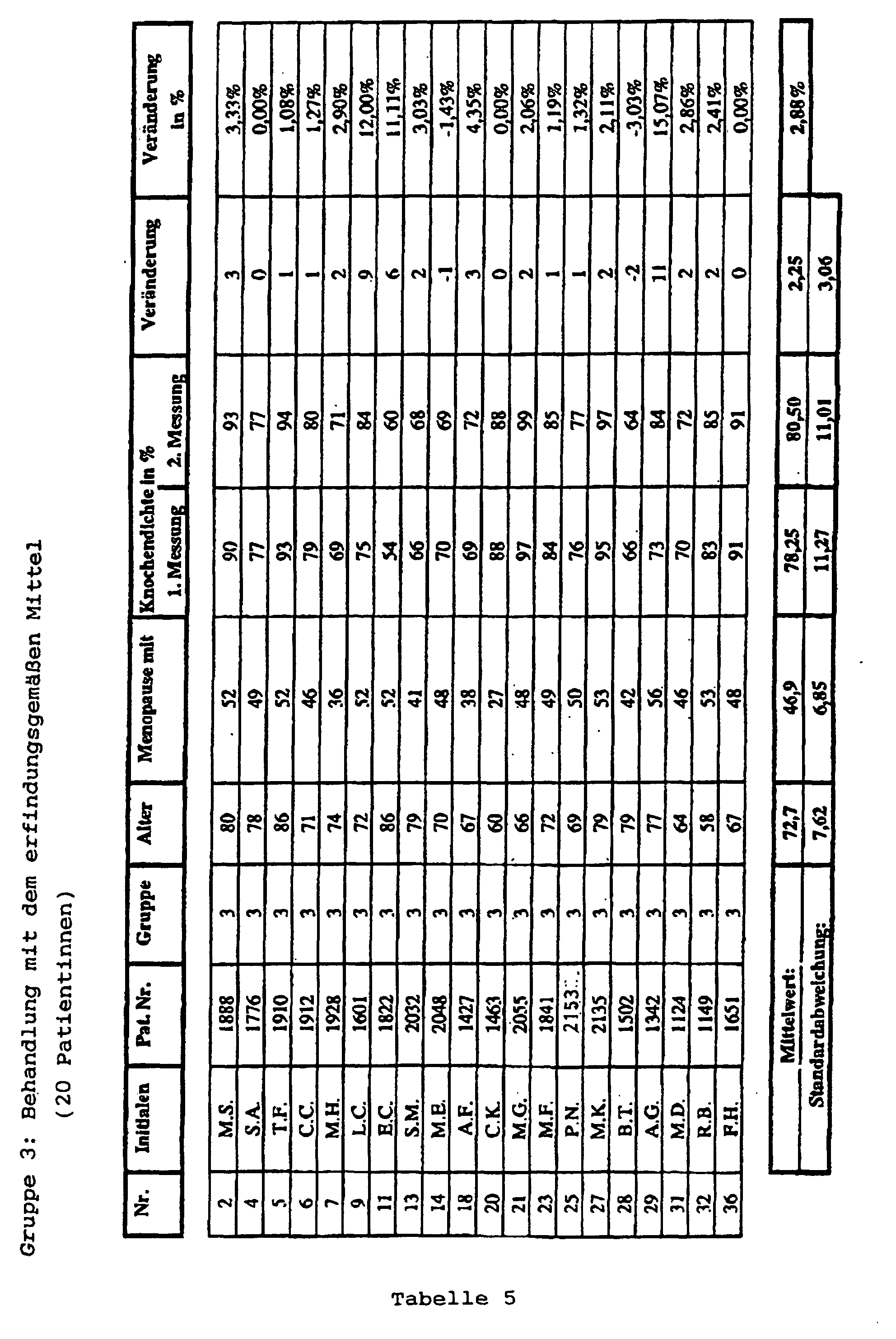

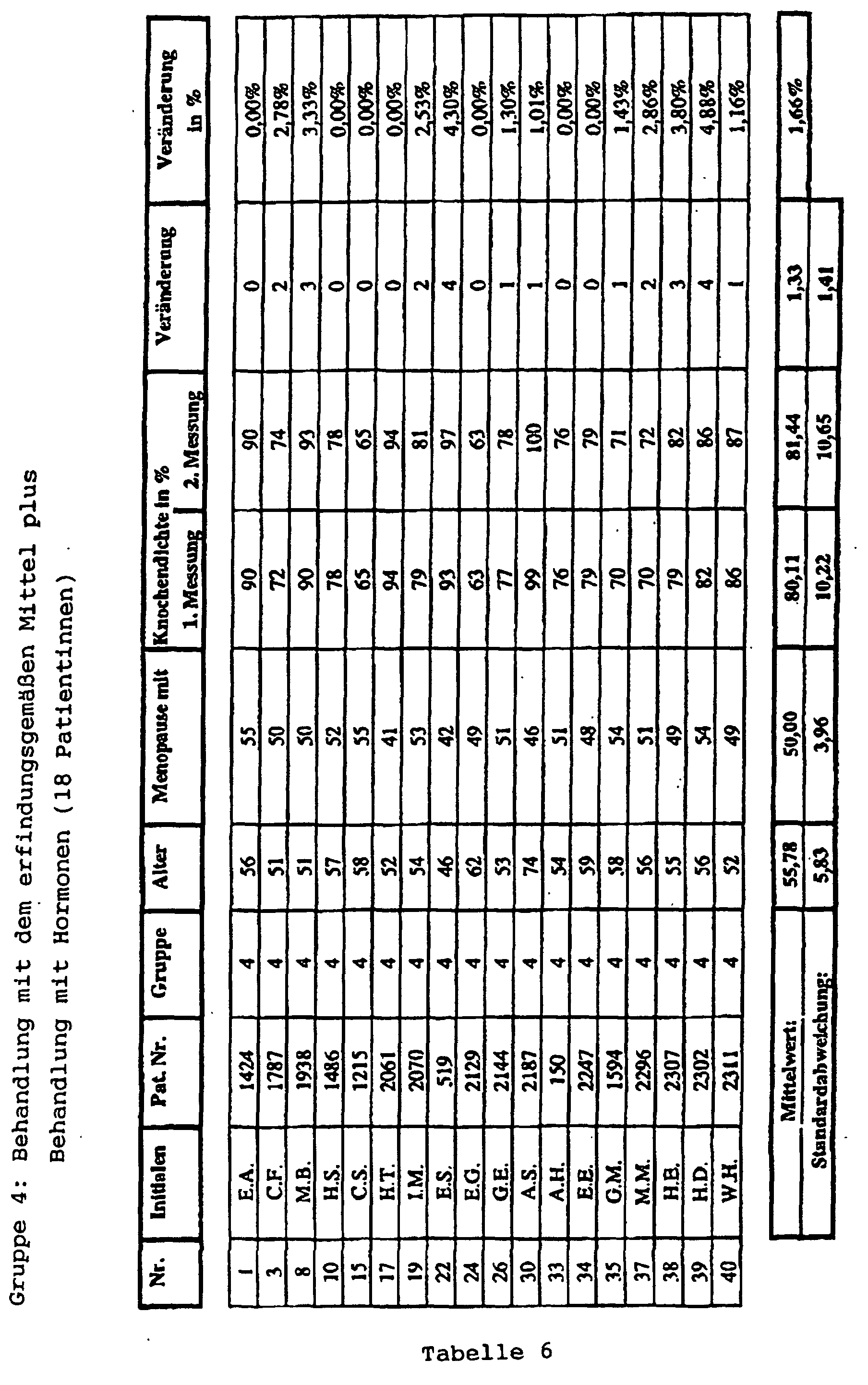

## Patentansprüche

1. Pharmazeutische oder diätetische Zusammensetzung umfassend
- Natrium-, Kalium-, Magnesium- und Calcium-Ionen, wobei zumindest Natrium und Kalium als Carbonate und/oder Bicarbonate vorliegen, und
- für den menschlichen Körper essentielle Mengen- und Spurenelemente, ausgewählt aus Li-, Sr-, Zn-, Fe-, Mn-, Cu-, Cr-, Mo-, Se-, F-Ionen, Vitaminen, vorzugsweise Vitamin C, K und D₃, sowie Mischungen derselben,
wobei das Masseverhältnis von K : Ca : Na : Mg 0,5-20 : 0,2-16 : 0,1-10 : 1 beträgt, die Zusammensetzung wenigstens 40 mmol an alkalischen oder alkalisierenden Ionen enthält und die in Wasser gelöste Zusammensetzung einen pH-Wert von ≥ 8,0 aufweist.

2. Zusammensetzung nach Anspruch 1, **dadurch gekennzeichnet, daß** sie Gerüststoffe, vorzugsweise auf Basis von Silizium, enthält.

3. Zusammensetzung nach Anspruch 1 oder 2, **dadurch gekennzeichnet, daß** sie Zusatz- oder Hilfsstoffe, vorzugsweise Kohlehydrate und/oder Aromastoffe, enthält.

4. Zusammensetzung nach Anspruch 3, **dadurch gekennzeichnet, daß** die Vitamine ausgewählt sind aus Vitamin C, K, D₃ oder Mischungen derselben.

5. Zusammensetzung nach einem der Ansprüche 1 bis 4, **dadurch gekennzeichnet, daß** das Masseverhältnis von K : Ca : Na : Mg 1-10 : 0,5-8 : 0,3-5 : 1 beträgt.

6. Zusammensetzung nach einem der Ansprüche 1 bis 5, **dadurch gekennzeichnet, daß** das Masseverhältnis von K : Ca : Na : Mg etwa 5 : 2,5 : 1,5 : 1 beträgt.

7. Zusammensetzung nach einem der Ansprüche 1 bis 6, **dadurch gekennzeichnet, daß** die in Wasser gelöste Zusammensetzung einen pH-Wert von 8,0 bis 9,5 aufweist.

8. Zusammensetzung nach einem der Ansprüche 1 bis 7, **dadurch gekennzeichnet, daß** die in Wasser gelöste Zusammensetzung einen pH-Wert von 8,9 bis 9,0 aufweist.

9. Zusammensetzung nach einem der Ansprüche 1 bis 8, **dadurch gekennzeichnet, daß** sie in einer pharmazeutisch verabreichbaren Formulierung vorliegt.

10. Zusammensetzung nach einem der Ansprüche 1 bis 8, **dadurch gekennzeichnet, daß** sie in einer lebensmitteltechnisch geeigneten Formulierung vorliegt.

11. Zusammensetzung nach einem der Ansprüche 1 bis 10 als Arzneimittel.

12. Verwendung einer Zusammensetzung nach einem der Ansprüche 1 bis 10 zur Herstellung einer Präparation zur Vorbeugung und Behandlung von azidotischen Erkrankungen.

13. Verwendung einer Zusammensetzung nach einem der Ansprüche 1 bis 10 zur Herstellung einer Präparation zur Vorbeugung und Behandlung von Osteoporose, insbesondere von primärer Osteoporose bzw. von generalisierter Osteoporose sowie zur Stimulierung der Knochenbildung (anorganische und organische Knochenmasse) nach Knochenfrakturen.

14. Verwendung einer Zusammensetzung nach einem der Ansprüche 1 bis 10 zur Herstellung einer Präparation zur Regeneration des Ionenhaushaltes, insbesondere in Bindegewebszellen.

15. Verwendung einer Zusammensetzung nach einem der Ansprüche 1 bis 10 zur Herstellung einer Präparation zur Stabilisierung und Verbesserung der Knochendichte, insbesondere der organischen Knochenmasse und der Knochenmineraldichte.

## Claims

1. A pharmaceutical or dietetic composition comprising
- sodium, potassium, magnesium and calcium ions, at least sodium and potassium being present as carbonates and/or bicarbonates, and
- mass and trace elements essential to the human body, selected from Li, Sr, Zn, Fe, Mn, Cu, Cr, Mo, Se, F ions, vitamins,
preferably vitamin C, K and D₃, as well as mixtures thereof, the quantitative ratio of K : Ca : Na : Mg being 0.5-20 : 0.2-16 : 0.1-10 : 1, the composition containing at least 40 mmol of alkaline or akalizing ions, and the composition dissolved in water having a pH of ≥ 8.0.

2. A composition according to claim 1, **characterised in that** it contains skeleton substances, preferably based on silicon.

3. A composition according to claim 1 or 2, **characterised in that** it contains additives or auxiliary ingredients, preferably carbohydrates and/or aromatics.

4. A composition according to claim 3, **characterised in that** the vitamins are selected from vitamin C, K, D₃ or mixtures thereof.

5. A composition according to any one of claims 1 to 4, **characterised in that** the mass ratio of K : Ca : Na : Mg is 1-10 : 0.5-8 : 0.3-5 : 1.

6. A composition according to any one of claims 1 to 5, **characterised in that** the mass ratio of K : Ca : Na : Mg is 5 : 2.5 : 1.5 : 1.

7. A composition according to any one of claims 1 to 6, **characterised in that** dissolved in water, the composition has a pH of from 8.0 to 9.5.

8. A composition according to any one of claims 1 to 7, **characterised in that** dissolved in water, the composition has a pH of from 8.9 to 9.0.

9. A composition according to any one of claims 1 to 8, **characterised in that** it is present in a pharmaceutically administrable formulation.

10. A composition according to any one of claims 1 to 8, **characterised in that** it is present in a formulation suitable in terms of food technology.

11. A composition according to any one of claims 1 to 10 as a medicament.

12. Use of a composition according to any one of claims 1 to 10 for producing a preparation for the prevention and treatment of acidotic diseases.

13. Use of a composition according to any one of claims 1 to 10 for producing a preparation for the prevention and treatment of osteoporosis, in particular primary osteoporosis or generalized osteoporosis, as well as for stimulating bone formation (inorganic and organic bone mass) after bone fractures.

14. Use of a composition according to any one of claims 1 to 10 for producing a preparation for the regeneration of the ion balance, in particular in connective tissue cells.

15. Use of a preparation according to any one of claims 1 to 10 for producing a preparation for stabilising and improving bone density, in particular the organic bone mass and the bone mineral density.

## Revendications

1. Composition pharmaceutique ou diététique comprenant
- des ions de sodium, de potassium, de magnésium et de calcium, au moins le sodium et le potassium étant présents en tant que carbonate et/ou bicarbonate et
- des substances minérales ainsi que des oligo-éléments essentiels au corps humain, choisis parmi des ions de Li, Sr, Zn, Fe, Mn, Cu, Cr, Mo, Se, F, des vitamines, de préférence les vitamines C, K et D₃, ainsi que leurs mélanges,
le rapport de masse de K : Ca : Na : Mg s'élevant à 0,5-20 : 0,2-16 : 0,1-10 : 1, la composition comprenant au moins 40 mmol d'ions alcalins ou alcalinisants, et la composition dissoute dans l'eau présentant une valeur pH de ≥ à 8,0.

2. Composition selon la revendication 1, **caractérisée en ce qu'**elle comprend des builders, de préférence sur la base de silicium.

3. Composition selon la revendication 1 ou 2, **caractérisée en ce qu'**elle comprend des additifs ou des adjuvants, de préférence des carbôhydrates et/ou des arômes.

4. Composition selon la revendication 3, **caractérisée en ce que** les vitamines sont choisies parmi les vitamines C, K, D₃ ou leurs mélanges.

5. Composition selon l'une des revendications 1 à 4, **caractérisée en ce que** le rapport de masse de K : Ca : Na : Mg s'élève à 1-10 : 0,5-8 : 0,3-5 : 1.

6. Composition selon l'une des revendications 1 à 5, **caractérisée en ce que** le rapport de masse de K : Ca : Na : Mg s'élève environ à 5 : 2,5 : 1,5 : 1.

7. Composition selon l'une des revendications 1 à 6, **caractérisée en ce que** la composition dissoute dans l'eau présente une valeur pH allant de 8,0 à 9,5.

8. Composition selon l'une des revendications 1 à 7, **caractérisée en ce que** la composition dissoute dans l'eau présente une valeur pH allant de 8,9 à 9,0.

9. Composition selon l'une des revendications 1 à 8, **caractérisée en ce qu'**elle est sous forme de formulation pour l'administration pharmaceutique.

10. , Composition selon l'une des revendications 1 à 8, **caractérisée en ce qu'**elle est sous forme de formulation adaptée dans un contexte alimentaire.

11. Composition selon l'une des revendications 1 à 10 en tant que médicament.

12. Utilisation d'une composition selon l'une des revendications 1 à 10 pour la production d'une préparation utile pour prévenir et traiter des maladies acidotiques.

13. Utilisation d'une composition selon l'une des revendications 1 à 10 pour la production d'une préparation utile pour prévenir et traiter l'ostéoporose, en particulier l'ostéoporose primaire ou, selon le cas, l'ostéoporose généralisée ainsi que pour stimuler l'ossification (masse osseuse inorganique et organique) après des fractures.

14. Utilisation d'une composition selon l'une des revendications 1 à 10, pour la production d'une préparation utile pour régénérer l'équilibre ionique, en particulier dans les cellules du tissu conjonctif.

15. Utilisation d'une composition selon l'une des revendications 1 à 10, pour la production d'une préparation utile pour stabiliser et d'améliorer la densité osseuse, en particulier la masse osseuse organique et de la densité minérale osseuse.
